# EUROPEAN PATENT SPECIFICATION

(11) **EP 1 658 294 B1**
(45) Date of publication and mention of the grant of the patent: **10.01.2007**
(21) Application number: 04764213.7
(22) Date of filing: 17.08.2004
(51) Int. Cl.: C07D 498/22, C07D 513/22, A61K 49/00

(54) **3H-PHENOXAZINE DERIVATIVES SUITABLE AS NEAR-INFRARED IMAGING AGENTS, PREPARATION AN USE THEREOF**
ALS MITTEL ZUR NAHINFRAROTABBILDUNG GEEIGNETE 3H-PHENOXAZINDERIVATE, DEREN HERSTELLUNG UND VERWENDUNG
DERIVES DE 3H-PHENOXAZINE UTILISES EN TANT QU'AGENTS D'IMAGERIE INFRAROUGE, LEUR PREPARATION ET LEUR UTILISATION

(30) Priority: 18.08.2003 US 495921 P
(43) Date of publication of application: 24.05.2006
(73) Proprietor: Novartis AG, 4056 Basel (CH); Novartis Pharma GmbH, 1230 Wien (AT)
(72) Inventor: AUBERSON, Yves, CH-4123 Allschwil (CH); GREMLICH, Hans-Ulrich, CH-4106 Therwil (CH); HINTERSTEINER, Martin, A-1130 Wien (AT); KINZY, Willy, 79540 Lörrach (DE); KNEUER, Rainer, 79539 Lörrach (DE)
(74) Representative: Roth, Peter Richard
(86) International application number: PCT/EP2004/009225
(87) International publication number: WO 2005/016934

(56) References cited:
- EP-A- 1 193 260
- WISCHIK C M ET AL: "SELECTIVE INHIBITION OF ALZHEIMER DISEASE-LIKE TAU AGGREGATION BY PHENOTHIAZINES" October 1996 (1996-10), PROCEEDINGS OF THE NATIONAL ACADEMY OF SCIENCES OF USA, NATIONAL ACADEMY OF SCIENCE. WASHINGTON, US, PAGE(S) 11213-11218 , XP002067057 ISSN: 0027-8424 the whole document
- H. MICHAEL PETRASSI ET AL..: "Structure-based Design of N-Phenyl Phenoxazine Transthyretin Amyloid Fibril Inhibitors" JOURNAL OF THE AMERICAN CHEMICAL SOCIETY, vol. 122, no. 10, 15 March 2000 (2000-03-15), pages 2178-2179, XP002306598 COLUMBUS OHIO

## Description

### Summary of the Invention

The invention relates to novel near-infrared imaging agents and the use of said agents in a method of labeling amyloid plaques in the brain. In particular, agents of the invention are useful in identifying amyloid formation and/or accumulation in neurological and vascular diseases such as Alzheimer's disease.

### Background of the Invention

Alzheimer's disease affects 8% of the population over 65, and at least 35% of those above the age of 80. No current method allows a definitive diagnosis of Alzheimer's disease before autopsy and clinicians can only make a diagnostic of probable Alzheimer's disease by comparing the results of several tests and observations leading to a diagnostic accuracy of about 80 to 90%. The identification of an imaging agent with enough specificity and sensitivity for Alzheimer's disease hallmarks would allow major advances in the way Alzheimer's disease is diagnosed.

Alzheimer's disease is a neurodegenerative disease of the brain characterized by dementia, cognitive impairment, and memory loss. The beta amyloid peptides Aβ1-40 and Aβ1-42 are major metabolites of the amyloid precursor protein and are found in senile plaques and cerebrovascular amyloid deposits in affected individuals. Formation and accumulation of beta amyloid peptides aggregates in the brain are considered to be critical factors in the development and progression of Alzheimer's disease. Another hallmark of the disease is hyperphosphorylation of the microtubule-associated protein tau with subsequent formation of neurofibrillary tangles.

Currently, the only definitive confirmation of Alzheimer's disease is by postmortem histopathological examination of amyloid deposits and neurofibrillary tangles in the brain. Early appraisal of clinical symptoms for diagnosis is often difficult and unreliable. Therefore, there is a need for in vivo imaging agents, which can specifically demonstrate the location and density of amyloid plaques and neurofibrillary tangles in the living brain. Such agents would be useful diagnostic tools for early detection and monitoring of disease progression, as well as for evaluating the effectiveness of treatments of Alzheimer's disease.

Neurofibrillary tangles are cytoskeletal elements composed of aggregates of hyper-phosphorylated tau proteins assembled into periodic restricted amyloid fibers in paired helical filaments. The major component of amyloid plaques is a 39-43 amino acid long beta-amyloid peptide that is generated from the cleavage of a larger amyloid precursor protein. Except for diffuse plaques formed almost exclusively of beta-amyloid peptides, amyloid plaques are complex lesions containing numerous associated cellular products. Deposits of beta-amyloid occur very early in the disease process long before the clinical symptoms develop.

The direct imaging of amyloid deposits in vivo is difficult as the deposits have many of the same physical properties (e.g. density and water content) as normal tissues. However, processes for irradiation and imaging of biological tissues with light of the wavelength range from 600 to 1000 nm in the near-infrared (NIR) region are available.

In fluorescence imaging, the energy from an external source of light, e.g. a laser is absorbed and almost immediately re-emitted at a longer wavelength of lower energy. Since biological tissue has a relatively high permeability for long-wave light of the 600 to 1000 nm spectral region, both the detection of non-absorbed radiation in the form of a transmission visualization and the re-emitted fluorescence radiation can provide tissue-specific data. Imaging of deeper tissues (that is, in the range of centimeters) is accomplished by using NIR light in combination with NIR fluorochromes. Near-infrared fluorescence imaging has the advantage of minimizing tissue autofluorescence thus improving target/background ratios.

Suitable agents for in vivo brain imaging should be able to cross the blood-brain barrier to enter the brain in sufficient amounts to be detectable by near-infrared radiation, be of low molecular weight and lipophilic. Further, they should have a high affinity and specific binding to amyloid plaques without being rapidly degraded.

### Description of the Invention

The present invention provides novel imaging agents that can be used in near-infrared imaging.

In one aspect of the invention, compounds of formula I 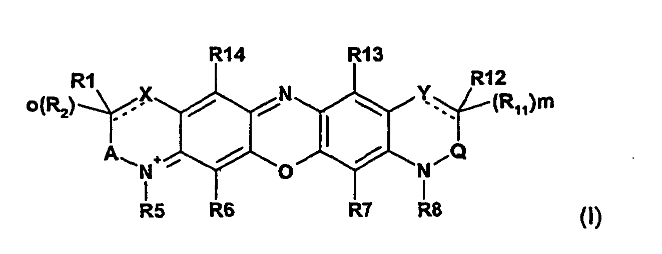 are provided in free base or acid addition salt form, wherein
X and Y represent CH, CH₂ or a divalent or trivalent heteroatom under the proviso that X and Y are not simultaneously CH or CH₂;
m and o represent independently of each other 0 or 1, with the proviso that
if m is 0 then the dotted line between Y and the neighboring C atom represents a bond and Y is CH or a trivalent heteroatom,
if m is 1 then the dotted line between Y and the neighboring C atom is absent and Y is CH₂ or a divalent heteroatom,
if o is 0 then the dotted line between X and the neighboring C atom represents a bond and X is CH or a trivalent heteroatom,
if o is 1 then the dotted line between X and the neighboring C atom is absent and X is CH₂ or a divalent heteroatom;
A represents (CR₃R₄)p and Q represents (CR₉R₁₀)ₙ;
n and p represent independently of each other 0 or 1;
R₆, R₇, R₁₃, and R₁₄ denote independently of each other hydrogen, halogen, (C₁₋₄)alkyl, (C₁₋₄)alkylSO₂, SO₃H, carboxy, (C₁₋₄)alkoxy carbonyl, (C₁₋₄)alkoxy, OH or NR₁₅R₁₆;
R₁, R₂, R₃, R₄, R₉, R₁₀, R₁₁ and R₁₂ denote independently of each other hydrogen, (C₁₋₄)alkyl, carboxy, (C₁₋₄)alkoxy carbonyl or (C₁₋₄)alkoxy, or, when X is C^{H} or CH₂ then R₁ and R₂ can also be OH or NR₁₅R₁₆, or when Y is CH or CH₂ then R₁₁, R₁₂ can also be OH or NR₁₅R₁₆;
**R₅,** R₈, R₁₅ and R₁₆ are independently of each other hydrogen, (C₁₋₄)alkyl, (C₁₋₄)alkoxy, R₁₇O-C(0)-(C₁₋₄)alkyl or (reactive group)-(C₁₋₄)alkyl; and
R₁₇ represents hydrogen or (C₁₋₄)alkyl.

In a preferred aspect of the invention, compounds of formula I are provided in free base or acid addition salt form wherein X is O, S or CH₂ and Y is O, S or CH₂, provided that X and Y are not both CH₂.

The compounds of formula bear a cationic charge by virtue of their structure and, hence, are always accompanied by an anion, e.g. an anion resulting from deprotonation of an anorganic or carboxylic acid, such as chloride, bromide, tetrafluoroborate or trifluoroacetate.

Above and elsewhere in the present description the following terms have the following meanings:

Hal or halogen denotes I, Br, Cl or F.

Organic radicals or compounds can be branched or unbranched.

An "alkyl group" is branched or unbranched and contains preferably 1 to 4 carbon atoms. Alkyl represents, for example, methyl, ethyl, propyl, butyl.

An "alkoxy group" is branched or unbranched and contains preferably 1 to 4 carbon atoms. Alkoxy represents for example methoxy, ethoxy, propoxy, butoxy.

"Alkyl carbonyl" refers to a radical of the formula -C(O)Rₐ where Rₐ is an alkyl radical as defined above.

### Short Description of the Figure

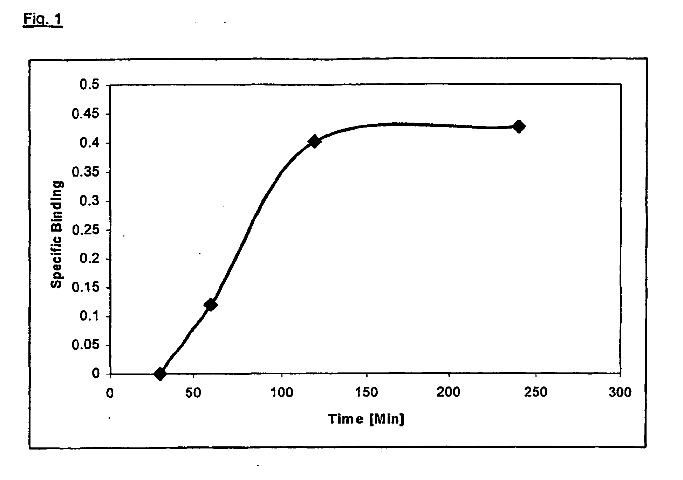

Fig. 1: The graph shows specific binding of the compound of Example 3 to AD plaques in female 16-months-old transgenic APP23 mice that are injected i.v. with 3 mg/kg agent of invention. Specific binding is defined as fluorescence signal(transgenic mice) minus fluorescence signal(non-transgenic mice) divided by fluorescence signal(transgenic mice).

In one aspect of the invention, compounds according to formula I are capable of being detected by near-infrared radiation of wavelength 600-1000 nm.

In yet another aspect of the invention, compounds of the invention preferably exhibit the following properties:
(i) specificity for amyloid plaques
(ii) blood-brain barrier penetration
(iii) solubility
(iv) capable of being detected by near-infrared radiation

Specificity of a compound of the invention for amyloid plaques is determined to have occurred when there is a chemical interaction between a compound of the invention and said amyloid plaque. This chemical association includes: covalent bonds, ionic bonds, hydrophilic-hydrophilic interactions or hydrophobic-hydrophobic interactions.

In one aspect of the invention, a composition is provided comprising a compound of formula I and a pharmaceutically acceptable excipient or diluent.

In yet another aspect of the invention, a composition is provided comprising a compound of the invention capable of being detected by near-infrared radiation of wavelength 600 -1000 nm.

Compositions according to the invention comprise a compound of the invention and are intended to include any and all solvents, dispersion media, coatings, isotonic and absorption delaying agents, and the like, compatible with application. Except insofar as any conventional media or agent is compatible with the active compound, such media can be used in the compositions of the invention. Supplementary active compounds can also be incorporated into the compositions.

For example, when a composition of the invention is applied to a subject, it is formulated to be compatible with its intended route of application. Examples of routes of application include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid or cyclodextrin; buffers such as acetates, citrates or phosphates and agents for the adjustment of tonicity such as sodium chloride or dextrose. pH can be adjusted with acids or bases, such as hydrochloric acid or sodium hydroxide.

Compounds or compositions of the invention can be attached to cells or vectors and be delivered to a subject by, for example, intravenous injection, local administration or by stereotactic injection.

Composition of the present invention may further include one or more of the following components, at the concentrations noted, with the final osmolality adjusted with sodium chloride or potassium chloride to from about 250 milliosmoles (m Osm) to about 330 milliosmoles: K/NaHCO 3 at a concentration of about 5-50 mM; MgCl 2 at a concentration of about 0-88 mM; KCI at a concentration of about 4-104 mM; Na 3 PO 4 at a concentration of about 0-1.5 mM; and CaCl 2 at a concentration of about 0- 0.6 mM. Preferably, the buffer solution is formulated to maintain the pH of the autoretic reagent composition at between about 7 to about 8, most preferably about 7.4, and, accordingly, may include one or more of the following components, in the concentration ranges given, with the final osmolality of from about 280 m Osm to about 300 m Osm: Tris/TEA at a concentration of about 0-150 mM; K 2 Ox/EDTA at a concentration of about 0-121 mM; and KCl/NaCl at a concentration of about 0-155 mM.

Compositions of the present invention may also include certain anions and cations (e.g., alkyl metal chlorides) to facilitate penetration through cell membranes. Non-limiting examples of anions include bicarbonate, chloride borate, barbital, oxalate (Ox), or ethylenediaminetetraacetic acid (EDTA). It is to be noted that not all anions have been found to be effective in promoting penetration across cell membranes. Nonlimiting examples of suitable cations include sodium (e.g., NaCl), potassium (e.g., KCI), trishydroxymethylamino methane (Tris), (Tris[hydroxymethyl]-aminomethane-hydrochloric acid (Tris-HCl), or triethanolamine (TEA).

In a further aspect of the invention, conjugates are provided comprising a compound of the invention covalently linked to a biomolecule through a reactive group. Biomolecules may be selected from the group consisting of: nucleoside, nucleotide, oligonucleotide, nucleic acid, protein, peptide, amino acid, polysaccharide, oligosaccharide, monosaccharide, drug or a small molecule, for example, less than MW 500.

The term "reactive group" as used herein means in particular a group as defined in Table 1.

| Table 1: Examples of reactive groups for covalent linkages | | |
|---|---|---|
| Reactive group | Coupled to | product |
| Activated ester (e.g. NHS) | Amine | amide |
| Sulfonyl halide | Phenol/alcohol | Sulfonate ester |
| Sulfonyl halide | Amine | Sulfonamide |
| Isothiocyanate | Amine | Thiourea |
| Anhydride | Amine | Amide |
| Maleimide | Thiol | Thioether |
| Thiol | Maleimide | Thioether |
| Haloacetyl | Thiol | Thioether |
| Hydrazine | Aldehyde | Hydrazone |
| Amine | Aldehyde | Amine (after reduction) |
| Amine | Reactive carboxylic acid | Amide |
| Phosphoramidite | Alcohol | Phosphite esters |
| Boronates | Glycols | Boronate esters |

In another aspect of the invention, a process for the production of compounds of formula I and their salts is provided, comprising the steps of reacting a phenol derivative of formula III 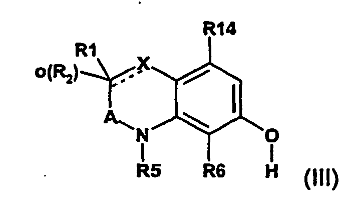 wherein the radicals and symbols A, X, R₁, R₂, R₅, R₆, R₁₄ and o have the meanings as defined in claim 1 for a compound of formula I, with a nitroso or diazo compound of formula IV 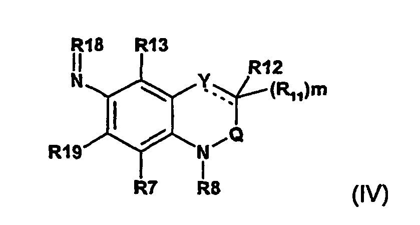 wherein the radicals and symbols Q, Y, R₇, R₈, R₁₁, R₁₂, R₁₃ and m have the meanings as defined in claim 1 for a compound of formula I, R₁₈ represents oxo or p-nitrophenyl-N= and R₁₉ represents hydroxy; and recovering the resulting compound of formula I in free base form or in form of an acid addition salt.

Compounds of the invention can be produced, for example, as described in Examples 1-5. Working up the reaction mixtures and purification of the compounds thus obtained may be carried out in accordance to known procedures. Acid addition salts may be produced from the free bases in known manner, and vice-versa. The starting materials of formula III and IV are known or can be prepared according to methods known in the art or those disdosed in the Examples.

The invention provides a method of labeling target structures in the brain by applying a compound or composition of the invention. The term "applying" is used in its broadest sense and includes any method of introducing the compounds or compositions of the invention to a subject's body or part of the body. A subject refers to any mammal including, for example, a human, rat, mouse, dog, cat or swine.

The application of a compound or composition of the invention to a subject may be systemically or locally. For example, a compound of the invention may be applied to the subject such that it is delivered throughout the body. Alternatively, a compound of the invention may be applied locally to a specific organ or tissue of interest. This local application may be either in vivo in a living subject or in vitro after a tissue sample has been removed from the body. Examples of the application of a compound or composition of the invention are described in the Examples section.

After a compound or composition of the invention is applied, detection of the compound using near-infrared radiation occurs. The amount of compound needed to be detected can readily be determined by those skilled in the art. Increasing the amount of compound of the invention and comparison to a suitable control can determine the precise dosage of compound needed.

The imaging of amyloid deposits can also be carried out quantitatively so that the amount of amyloid deposits can be determined. For quantitative analysis, the fluorescence images are analyzed on a region of interest basis.

In an aspect of the invention, a method of labeling target structures in the brain is provided, comprising:
(i) applying a composition comprising a compound of formula I in free base or acid addition salt form
(ii) allowing sufficient time for said compound to be chemically associated with the target structure in the brain
(iii) detecting said compound using near-infrared radiation.

In another aspect of the invention, a method of labeling target structures in the brain is provided comprising:
(i) applying a composition comprising a compound of formula I, wherein X is O,S, or C and Y is O, S or CH₂ with the proviso that X and Y are not both CH₂
(ii) allowing sufficient time for said compound to be chemically associated with the target structure in the brain and
(iii) detecting said compound using near-infrared radiation.

In yet another aspect of the invention, a method of labeling target structures in the brain is provided comprising:
(i) applying a composition comprising a compound of formula 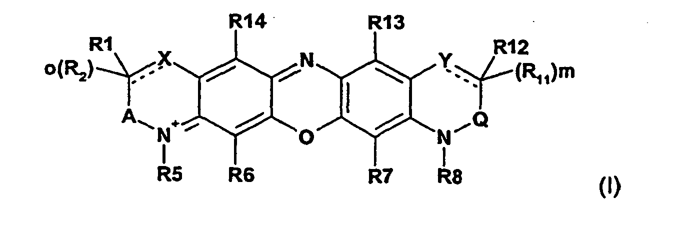 wherein
   X and Y represent CH, CH₂ or a divalent or trivalent heteroatom under the proviso that X and Y are not simultaneously CH or CH₂;
   m and o represent independently of each other 0 or 1, with the proviso that
   if m is 0 then the dotted line between Y and the neighboring C atom represents a bond and Y is CH or a trivalent heteroatom,
   if m is 1 then the dotted line between Y and the neighboring C atom is absent and Y is CH₂ or a divalent heteroatom,
   if o is 0 then the dotted line between X and the neighboring C atom represents a bond and X is CH or a trivalent heteroatom,
   if o is 1 then the dotted line between X and the neighboring C atom is absent and X is CH₂ or a divalent heteroatom;
   A represents (CR₃R₄)ₚ and Q represents (CR₉R₁₀)ₙ;
   n and p represent independently of each other 0 or 1;
   R₆, R₇, R₁₃, and R₁₄ denote independently of each other hydrogen, halogen, (C₁₋₄)alkyl, (C₁₋₄)alkylSO₂, SO₃H, carboxy, (C₁₋₄)alkoxy carbonyl, (C₁₋₄)alkoxy, OH or NR₁₅R₁₆;
   R₁, R₂, R₃, R₄, R₉, R₁₀, R₁₁ and R₁₂ denote independently of each other hydrogen, (C₁₋₄)alkyl, carboxy, (C₁₋₄)alkoxy carbonyl or (C₁₋₄)alkoxy, or, when X is CH or CH₂ then R₁ and R₂ can also be OH or NR₁₅R₁₆, or when Y is CH or CH₂ then R₁₁, R₁₂ can also be OH or NR₁₅R₁₆;
   R₅, R₈, R₁₅ and R₁₆ are independently of each other hydrogen, (C₁₋₄)alkyl, (C₁₋₄)alkoxy, R₁₇O-C(O)-(C₁₋₄)alkyl or (reactive group)-(C₁₋₄)alkyl; and
   R₁₇ represents hydrogen or (C₁₋₄) alkyl;
   in free base or acid addition salt form, or of formula II 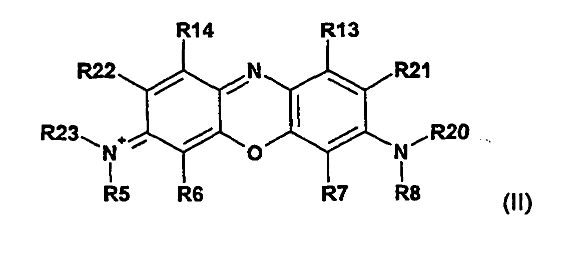 wherein
   R₆, R₇, R₁₃, and R₁₄ denote independently of each other hydrogen, halogen, (C₁₋₄)alkyl, (C₁₋₄)alkylSO₂, SO₃H, carboxy, (C₁₋₄)alkoxy carbonyl, (C₁₋₄)alkoxy, OH or NR₁₅R₁₆, and
   R₂₁ and R₂₂ are hydrogen,(C₁₋₄)alkyl, (C₁₋₄)alkoxy, phenyl, phenylalkyl, carboxy or halogen;
   R₁₄ and R₂₂ together with the carbon atoms to which they are attached can also form a saturated or unsaturated ring;
   R₂₁ and R₁₃ together with the carbon atoms to which they are attached can also form a saturated or unsaturated ring;
   R₅, R₈, R₂₀ and R₂₃ are hydrogen, (C₁₋₄)alkyl, (C₁₋₄)alkoxy, polyoxyhydrocarbyl, phenyl, phenylalkyl;
   R₈ and R₂₀ together with the nitrogen atom to which they are attached can form a saturated or unsaturated ring,
   R₂₃and R₅ together with the nitrogen atom to which they are attached can form a saturated or unsaturated ring,
   R₂₂and R₂₃ together with the atoms to which they are attached can form a saturated or unsaturated ring,
   R₅ together with R₆ together with the atoms to which they are attached can form a saturated or unsaturated ring,
   R₇ together with R₈ together with the atoms to which they are attached can form a saturated or unsaturated ring,
   R₂₀ together with R₂₁ together with the atoms to which they are attached can form a saturated or unsaturated ring,
(ii) allowing sufficient time for said compound to be chemically associated with the target structure in the brain, and
(iii) detecting said compound using near-infrared radiation.

Compounds and compositions of the invention are useful as markers for labeling pathological structures such as amyloid plaques in the brain. This is useful in identifying, diagnosing and preventing diseases involving the formation and/or accumulation of amyloid plaques and for monitoring the effectiveness of therapeutic treatments of diseases involving formation and/or accumulation of amyloid plaques. These diseases include, for example, Alzheimer's disease, Down 's syndrome, memory and cognitive impairment, dementia, amyloid neuropathies, brain inflammation, nerve and brain trauma, vascular amyloidosis, or cerebral hemorrhage with amyloidosis.

In another aspect of the invention, compounds and compositions of the invention are used as near-infrared imaging agents for identifying amyloid plaques in the brain.

The following examples are illustrative, but not limiting, of the method and compositions of the present invention. Other suitable modifications and adaptations of the variety of conditions and parameters normally encountered and obvious to those skilled in the art are within the spirit and scope of the invention.

### Example 1: Synthesis of 4,8-Dimethyl-2,3,4,9,10,11-hexahydro-1,6-dioxa-4,13-diaza-8-azonia-pentacen chloride

A solution of 1-methyl-1,2,3,4-tetrahydro-quinolin-7-ol (20.0 mg, 123 µmol) in water (500 µl) and 2 M HCl (100 µl) is cooled to 0°C and an aqueous solution of sodium nitrite (8.57 mg, 123 µmol) is added. The reaction mixture is stirred at 0°C for 60 min, neutralized with a saturated solution of NaHCO₃ and extracted with ethyl acetate. The organic phase is dried with MgSO₄ and the solvent is removed under reduced pressure. The obtained nitroso intermediate and 4-methyl-2-H-benz[1,4]oxazin-6-ol (24.5 mg, 148 µmol) are dissolved in a mixture of ethanol (1.00 ml) and 2 M HCl (100 µl) and heated under reflux for 1 h. The solution is concentrated under reduced pressure and the residue is purified by column chromatography (SiO₂, dichloromethane/methanol = 10/3) providing the title compounds as blue crystals, mp: 245-248 °C; ¹H NMR (500 MHz): δ = 2.05 (tt, 2 H, *³J=* 6.1 Hz, *³J* = 5.5 Hz, 10-H), 2.92 (t, 2 H, ³*J* = 6.1 Hz, 11-H), 3.31 (s, 3 H, 1'-H), 3.33 (s, 3 H, 2'-H), 3.70 (t, 2 H, ³*J* = 5.5 Hz, 9-H), 3.78 (t, 2-H, ³*J* = 4.9 Hz, 3-H), 4.35 (t, 2 H, ³*J* = 4.9 Hz, 2-H), 6.82 (s, 1H, 7-H), 6.90 (s, 1 H, 5-H), 7.09 (s, 1 H, 11-H), 7.40 (s, 1 H, 12-H).

### Example 2: Synthesis of 8-Ethyl-4-methyl-2,3,4,9,10,11-hexahydro-1,6-dioxa-4,13-diaza-8-azonia-pentacen chloride

A solution of 1-ethyl-1,2,3,4-tetrahydro-quinolin-7-ol (50.0 mg, 276 µmol) in water (1 ml) and 2 M HCl (250 µl) is cooled to 0°C and a solution of sodium nitrite (20.0 mg, 287 µmol) in water (400 µl) is added. The reaction mixture is stirred at 0°C for 60 min, neutralized with a saturated solution of NaHCO₃ and extracted with ethyl acetate. The organic phase is dried with MgSO₄ and the solvent is removed under reduced pressure. The nitroso intermediate (25.0 mg, 121 µmol) and 4-methyl-2-H-benz[1,4]oxazin-6-ol (20.1 mg, 121 µmol) are dissolved in a mixture of ethanol (750 µl) and 2 M HCl (124 µl) and heated under reflux for 3 h. The solution is concentrated under reduced pressure and the residue is purified by column chromatography (SiO₂, dichloromethane/methanol/water/ acetic acid = 10/10/1/1) to furnish the title compound as blue crystals, mp: 245-247 °C; ¹H NMR (500 MHz): δ = 1.24 (t, 3 H, ³*J*= 6.5 Hz, 2'-H), 1.94 (m, 2 H, 10-H), 2.89 (m, 2 H, 11-H), 3.34 (s, 3 H, 3'-H), 3.67 (m, 2 H, 9-H), 3.73 (m, 2H,1'-H), 3.79 (m, 2 H, 3-H), 4.34 (m, 2 H, C-2), 6.99 (s, 1 H, 7-H), 7.01 (s, 1 H, 5-H), 7.22 (s, 1 H, 14-H), 7.55 (s, 1 H, 6-H).

### Example 3: 4,8-Dimethyl-3,8,9,10-tetrahydro-2H-1,6,11-trioxa-8,13-diaza-4-azonia-pentacen tetrafluoroborate

4-Nitro-benzenediazonium tetrafluoroborate (574 mg, 2.42 mmol) is dissolved in 10% H₂SO₄ (400 µl) and is added to a solution of 4-methyl-2-H-benz[1,4]oxazin-6-ol (400 mg, 2.42 mmol) in methanol (2 ml). The reaction mixture is stirred for 30 min at room temperature, neutralized with 25 % aqueous ammonia and the red precipitate is spun down. The crude diazo intermediate is purified by recrystallization (n-butanol); mp: 162-165 °C.

The diazo intermediate (560 mg, 1.78 mmol) and 4-methyl-2-H-benz[1,4]oxazin-6-ol (326 mg, 1.96 mmol) are dissolved in a mixture of ethanol (10 ml) and water (1 ml). After the addition of 32 % HCl (700 µl) the reaction mixture is stirred at 70°C under reflux for 1 h and subsequently the solution is concentrated under reduced pressure. The residue is dissolved in water and treated with a saturated solution of sodium tetrafluoroborate. The precipitate is spun down and purified by column chromatography (SiO₂, dichloromethane/methanol = 10/2) to provide the title compound as blue crystals, mp: 235-238 °C.
¹H NMR (500 MHz): δ = 3.37 (s, 6 H, 1'-H, 2'-H), 3.80 (t, 4 H, ³*J* = 4.8 Hz, 3-H, 9-H), 4.37 (t, 4 H, ³*J* = 4.8 Hz, 2-H, 10-H), 7.03 (s, 2 H, 5-H, 7-H), 7.21 (s, 2 H, 12-H, 14-H).

### Example 4: 4,8-Dimethyl-2,3,9,10-tetrahydro-4H-1,6-dioxa-11-thia-4,13-diaza-8-azonia-pentacen chloride

4-Nitro-benzenediazonium tetrafluoroborate (287 mg, 1.21 mmol) is dissolved in 10 % H₂SO₄ (200 µl) and is added to a solution of 4-methyl-3,4-dihydro-2H-benzo[1,4]thiazin (200 mg, 1.21 mmol) in methanol (2 ml). The reaction mixture is stirred for 30 min at room temperature, neutralized with 25 % aqueous ammonia and the red precipitate is spun down. The crude diazo intermediate is used in the next stage without further purification.
The diazo intermediate (150 mg, 477 µmol) and 4-methyl-2-H-benz[1,4]oxazin-6-ol (119 mg, 716 µmol) are dissolved in a mixture of ethanol (4 ml) and water (400 µl). After the addition of 32 % HCl (187 µl) the reaction mixture is heated at 70°C under reflux for 1 h and subsequently the solution is concentrated under reduced pressure. The crude product is purified by column chromatography (SiO₂, dichloromethane/methanol = 10/3) to yield: the title compound as blue crystals; mp: 250-252 °C.
¹H NMR (500 MHz): δ = 3.17 (t, 2 H, ³*J* = 4.9 Hz, 10-H), 3.37 (s, 3 H, 1'-H), 3.41 (s, 3 H, 2'-H), 3.85 (m, 2 H, 9-H), 4.03 (m, 2 H, 3-H), 4.38 (d, 2 H,³*J* = 4.3 Hz, 2-H), 6.99 (s, 1 H, 7-H), 7.02 (s, 1 H, 5-H), 7.11 (s, 1 H, 14-H), 7.54 (s, 1 H, 12-H).

### Example 5: 8-(3-Ethoxycarbonyl-propyl)-4-methyl-3,8,9,10-tetrahydro-2H-1,6,11-trioxa-8,13-diaza-4-azonia-pentacen chloride

A solution of 3,4-dihydro-2H-1,4-benzoxazin-6-ol (200 mg, 1.32 mmol), potassium carbonate (183 mg, 1.32 mmol) and 4-bromo-butyric acid ethyl ester (217 µl, 1.46 mmol) in anhydrous dimethyl formamide (1 ml) is stirred at 65°C for 16 h under argon. The reaction mixture is poured into water (20 ml), the aqueous phase is neutralized with 2 M HCl and extracted with ethyl acetate (3 x 50 ml). The combined organic phases are dried with MgSO₄ and the solvent is removed under reduced pressure. The crude product is purified by column chromatography (SiO₂, hexane/ethyl acetate = 2/1) furnishing a colorless oil; ¹H NMR (500 MHz): δ = 1.17 (t, 3 H, ³*J* = 7.4 Hz, 7'-H), 1.76 (dd, 2 H, ³*J* = 7.4 Hz, 2'-H), 2.34 (t, 2 H, ³*J* = 7.3 Hz, 3'-H), 3.17 (t, 2 H, ³*J* = 7.4 Hz, 1'-H), 3.23 (d, 2 H, ³*J* = 4.5 Hz, 3-H), 4.05 (m, 4 H, 2-H, 6'-H), 5.89 (dd, 1 H, ³*J* = 7.9 Hz, ⁴*J* = 2.4 Hz, 7-H), 6.11 (d, 1 H, ⁴*J* = 2.4 Hz, 5-H), 6.42 (d, 1 H, ³*J* = 7.9 Hz, 8-H), 8.62 (s, 1 H, OH).

The diazo intermediate from Example 3 (150 mg, 565 µmol) and 4-(6-hydroxy-2,3-dihydrobenzo[1,4]oxazin-4-yl)-butyric acid ethyl ester (213 mg, 678 µmol) are dissolved in a mixture of ethanol (1 ml) and water (100 µl). After the addition of 32 % HCl (222 µl) the reaction mixture is heated at 70°C under reflux for 1 h and the solution is concentrated under reduced pressure. The residue is purified by column chromatography (SiO₂, dichloromethane/methanol = 10/2) furnishing the title compound as blue crystals, mp: 242-245 °C.

### Example 6: UV/VIS and fluorescence properties of compounds of the invention

UV/VIS and fluorescence spectra are recorded and analyzed. For UV/VIS spectra, a Lambda 19 spectrometer from Perkin Elmer equipped with cells of 1.000 cm pathlength is used. The scan rate is 120 nm/min. The fluorescence data are obtained on a Spex Fluorolog (I.S.A.) spectrometer equipped with a cooled R928 detector (Slit 1 mm). Quantum yields are determined by using Cresylviolett (exc. 595 nm, yield = 0.54) as standard.

**Table 2: Fluorescent properties of compounds of the invention**

| Compound | solvent | Conc. | Absorption | | Fluorescence | |
|---|---|---|---|---|---|---|
| Example | | [M/I] | λₘₐₓ [nm] | ε [I/Mcm] | λₘₐₓ [nm] | φ [%] |
| Ex. 2 | methanol | 1.344E-6 | 649 | 67430 | 677 | 61 |
| Ex. 2 | H₂O | 1.706E-6 | 657 | 56700 | 677 | 24 |
| Ex. 2 | mouse serum | 1.706E-6 | 658 | - | 677 | 28 |
| Ex. 3 | methanol | 1.526E-6 | 644 | 64570 | 670 | 61 |
| Ex. 3 | H₂O | 1.531E-6 | 650 | 61930 | 670 | 37 |
| Ex. 3 | mouse serum | 1.531 E-6 | 650 | - | 670 | 41 |
| Ex. 4 | methanol | 6.706E-6 | 659 | 13920 | 695 | 28 |
| Ex. 4 | H₂O | 5.828E-6 | 665 | 12880 | 695 | 12 |
| Ex. 4 | mouse serum | 5.828E-6 | 665 | - | 695 | 13 |

### Example 7: Labeling of APP23 mouse and human Alzheimer disease (AD) brain sections using a compound of the invention or Thioflavine S

Four-micrometer thick paraffin sections from an APP23 mouse at 26 months of age are deparaffinized in xylene and rehydrated. 10 mg of the agent of the invention (compound Ex.3) is dissolved in 1 ml dimethylsulfoxide and diluted with deionized water 1:10. This staining solution is applied on sections for about 20 min. Section background is cleared by washing with 95% ethanol. Finally, sections are dehydrated in 99% ethanol, cleared in xylene and mounted with Vectashield^{™}. Twenty-micrometer thick cryotome sections from an AD brain cortex are air-dried and fixated in 4% PFA for 5 min. After washing in tap water sections are stained either with Thioflavine S or with the agent of the invention (compound Ex. 3) for 5 min and further processed as described above. The agent of the invention is dissolved in dimethylsulfoxide and diluted to a final concentration of 0.01 % with 50% Ethanol, Thioflavine S is dissolved in 50% Ethanol, final concentration is 0.01 %.

### Example 8: In vitro binding properties to amyloid plaques

Binding of the agent of the invention (compound Ex.3) to aggregated Aβ peptide Aβ1-40 in solution is investigated by differential fluorescence spectroscopy and thioflavine displacement. The fluorescence spectrum of the agent of the invention (compound Ex.3) shows slightly reduced fluorescence intensity in the range of 680-720 nm upon β-amyloid binding. Difference spectra are recorded at different concentrations of the agent of the invention (compound Ex.3) with the magnitude of the fluorescence shift being dependent on the concentration of aggregated Aβ. Saturation is observed at high concentrations of the agent of the invention (compound Ex.3), and the Kd-value for the agent of the invention (compound Ex.3) is estimated to be 0.2 µM. In another experiment, aggregated Aβ is incubated with thioflavine T, where fluorescence intensity at 460-560 nm (excitation 445 nm) represents thioflavine bound to aggregated Aβ. Addition of the agent of the invention (compound Ex.3) causes a concentration-dependent fluorescence reduction, indicating a displacement of thioflavine from aggregated Aβ, and the apparent binding constant for the agent of the invention (compound Ex.3) can be calculated. The experiment is done at thioflavine concentrations from 0.37 µM - 3 µM, the apparent binding constants for the agent of the invention (compound Ex.3) are between 0.1 µM - 0.2 µM at all thioflavine concentrations and do not increase at higher thioflavine concentrations.

### Example 9: In vivo labeling of Aβ in APP23 mice

Injection solution is prepared fresh by dissolving 10 mg of the agent of invention in 0.2 mL dimethylsulfoxide diluted with 9.8 ml sterile water. Lower concentrations are prepared by further dilution with water. Four APP23 female mice at 21 month of age receive one single injection of the compound (Injection volume: 1 ml/100gr body weight). The treated animals are killed by decapitation after one hour. The brains are removed and frozen on dry ice. 14 µm thick sections are cut in a cryotome, thaw mounted and air-dried. Staining is performed as described above. Sections are analyzed using conventional fluorescence microscopy and confocal microscopy.
(i) Staining of APP23 mice brain sections (which contain amyloid deposits but no neurofibrillary tangles): The agents of the invention strongly stain amyloid plaques and vascular amyloid deposits in brain sections of APP23 mice.
(ii) Staining of human AD brain sections (which contain both amyloid deposits and neurofibrillary tangles): Brain sections taken from frontal cortex of AD patients are stained with the agents of the invention, and the results compared with a Thioflavine S stain. The agents of the invention intensely and selectively stain amyloid deposits.
(iii) Ex vivo staining in APP23 mice: Intravenous administration of the agents of the invention in APP23 mice leads to a selective and intense staining of amyloid deposits, analyzed ex vivo.

### Example 10: Label and real-time detection of beta amyloid plaques and neurofibrillary tangles in vivo using near-infrared imaging

For in vivo near-infrared imaging, 10- to 24-months-old transgenic APP23 mice (3 ≤ n ≤ 5) and as control, non-transgenic APP23 mice of the same age (3 ≤ n ≤ 4) are injected intravenously (i.v.) into the tail-vein with 0.1, 1 and 3 mg/kg agent of invention (0.1, 1 and 3 mg/10 ml in 0.9% saline). Images are recorded 30, 60, 120 and 240 min after systemic administration. The graph in Fig. 1 shows specific binding of the compound of Example 3 to AD plaques in female 16-months-old transgenic APP23 mice that are injected i.v. with 3 mg/kg agent of invention. Here, specific binding is defined as fluorescence signal (transgenic mice) minus fluorescence signal (non-transgenic mice) divided by fluorescence signal (transgenic mice).
Significant differences in fluorescence signal intensity, i.e. specific binding is observed 60, 120 and 240 min after administration of the agent of invention in transgenic APP23 and non-transgenic control mice thus demonstrating the capability of the agent of invention for labeling amyloid plaques in the brain in order to identify Alzheimer's disease.

## Claims

1. Compounds of formula I 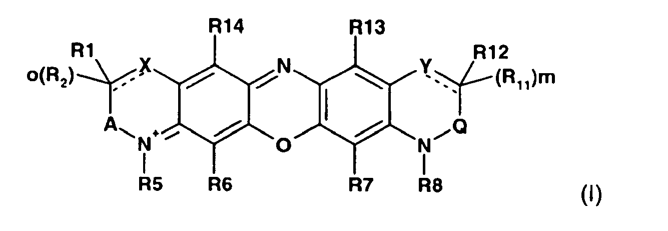 wherein
X and Y represent CH, CH₂ or a divalent or-trivalent heteroatom under the proviso that X and Y are not simultaneously CH or CH₂;
m and o represent independently of each other 0 or 1, with the proviso that
if m is 0 then the dotted line between Y and the neighboring C atom represents a bond and Y is CH or a trivalent heteroatom,
if m is 1 then the dotted line between Y and the neighboring C atom is absent and Y is CH₂ or a divalent heteroatom,
if o is 0 then the dotted line between X and the neighboring C atom represents a bond and X is CH or a trivalent heteroatom,
if o is 1 then the dotted line between X and the neighboring C atom is absent and X is CH₂ or a divalent heteroatom;
A represents (CR₃R₄)ₚ and Q represents (CR₉R₁₀)ₙ;
n and p represent independently of each other 0 or 1;
R₆, R₇, R₁₃, and R₁₄ denote independently of each other hydrogen, halogen, (C₁₋₄)alkyl, (C₁-₄)alkylSO₂, SO₃H, carboxy, (C₁₋₄)alkoxy carbonyl, (C₁₋₄)alkoxy, OH or NR₁₅R₁₆;
R₁, R₂, R₃, R₄, R₉, R₁₀, R₁₁ and R₁₂ denote independently of each other hydrogen, (C₁₋₄)alkyl, carboxy, (C₁₋₄)alkoxy carbonyl or (C₁₋₄)alkoxy, or, when X is CH or CH₂ then R₁ and R₂ can also be OH or NR₁₅R₁₆, or when Y is CH or CH₂ then R₁₁, R₁₂ can also be OH or NR₁₅R₁₆;
R₅, R₈, R₁₅ and R₁₆ are independently of each other hydrogen, (C₁₋₄)alkyl, (C₁₋₄)alkoxy, R₁₇O-C(O)-(C₁₋₄)alkyl or (reactive group)-(C₁₋₄)alkyl; and
R₁₇ represents hydrogen or (C₁₋₄)alkyl;
in free base or acid addition salt form.

2. A compound of formula I according to claim 1 in free base or acid addition salt form wherein X is O, S or CH₂ and Y is O S or CH₂ with the proviso that X and Y are not both simultaneously CH₂.

3. A compound of formula I according to claim 1 wherein said compound is selected from 4,8-dimethyl-2,3,4,9,10,11-hexahydro-1,6-dioxa-4,13-diaza-8-azonia-pentacen chloride; 8-ethyl-4-methyl-2,3,4,9,10,11-hexahydro-1,6-dioxa-4,13-diaza-8-azonia-pentacen chloride; 4,8-dimethyl-3,8,9,10-tetrahydro-2H-1,6,11-trioxa-8,13-diaza-4-azonia-pentacen tetrafluoroborate;
4,8-dimethyl-2,3,9,10-tetrahydro-4H-1,6-dioxa-11-thia-4,13-diaza-8-azonia-pentacen chloride; and
8-(3-ethoxycarbonyl-propyl)-4-methyl-3,8,9,10-tetrahydro-2H-1,6,11-trioxa-8,13-diaza-4-azonia-pentacen chloride.

4. A composition comprising a compound according to of any one of claims 1-3 and a pharmaceutically acceptable excipient or diluent.

5. A process for the production of a compound of formula I or a salt thereof, comprising the steps of reacting a phenol derivative of formula III 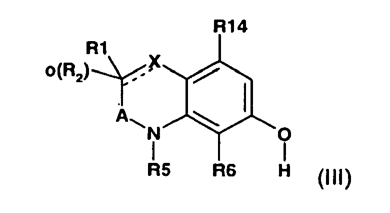 wherein the radicals and symbols A, X, R₁, R₂, R₅, R₆, R₁₄ and o have the meanings as defined in claim 1 for a compound of formula I, with a nitroso or diazo compound of formula IV 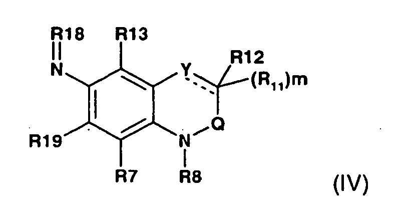 wherein the radicals and symbols Q, Y, R₇, R₈, R₁₁, R₁₂, R₁₃ and m have the meanings as defined in claim 1 for a compound of formula I, R₁₈ represents oxo or p-nitrophenyl-N= and R₁₉ represents hydroxy;
and recovering the resulting compound of formula I in free base form or in form of an acid addition salt.

6. Use of a compound of formula according to any one of claims 1-3 in free base or acid addition salt form as a near-infrared imaging agent.

7. Use of a compound of formula II 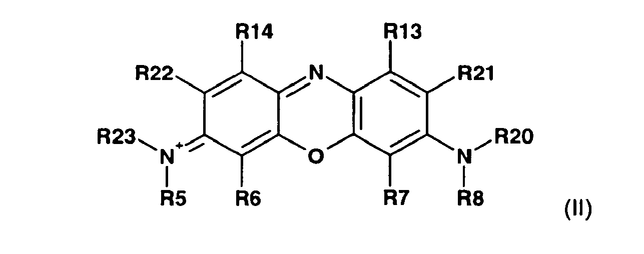 wherein
R₆, R₇, R₁₃, and R₁₄ denote independently of each other hydrogen, halogen, (C₁₋₄)alkyl, (C₁-₄)alkylSO₂, SO₃H, carboxy, (C₁₋₄)alkoxy carbonyl, (C₁₋₄)alkoxy, OH or NR₁₅R₁₆, and
R₂₁ and R₂₂ are hydrogen,(C₁₋₄)alkyl, (C₁₋₄)alkoxy, phenyl, phenylalkyl, carboxy or halogen;
R₁₄ and R₂₂ together with the carbon atoms to which they are attached can also form a saturated or unsaturated ring;
R₂₁ and R₁₃ together with the carbon atoms to which they are attached can also form a saturated or unsaturated ring;
R₅, R₈, R₂₀ and R₂₃ are hydrogen, (C₁₋₄)alkyl, (C₁₋₄)alkoxy, polyoxyhydrocarbyl, phenyl, phenylalkyl;
R₈ and R₂₀ together with the nitrogen atom to which they are attached can form a saturated or unsaturated ring,
R₂₃ and R₅ together with the nitrogen atom to which they are attached can form a saturated or unsaturated ring,
R₂₂ and R₂₃ together with the atoms to which they are attached can form a saturated or unsaturated ring,
R₅ together with R₆ together with the atoms to which they are attached can form a saturated or unsaturated ring,
R₇ together with R₈ together with the atoms to which they are attached can form a saturated or unsaturated ring,
R₂₀ together with R₂₁ together with the atoms to which they are attached can form a saturated or unsaturated ring,
as defined in claim 6 as a near-infrared imaging agent.

8. Use according to claims 6 or 7 as a near-infrared imaging agent to image amyloid plaques.

9. A conjugate comprising a compound of formula I according to any one of claims 1-3 covalently linked to a biomolecule through a reactive group.

10. A conjugate according to claim 9 wherein the biomolecule is selected from the group consisting of nucleoside, nucleotide, oligonucleotide, nucleic acid, protein, peptide, amino acid, polysaccharide, oligosaccharide, monosaccharide, drug or a small molecule having a molecular weight of less than 500.

11. A conjugate according to claim 9 or 10 capable of being detected using near-infrared radiation.

## Patentansprüche

1. Verbindungen der Formel I 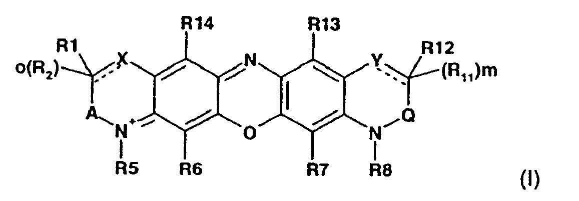 worin
X und Y für CH, CH₂ oder ein divalentes oder trivalentes Heteroatom stehen, mit der Maßgabe, dass X und Y nicht zugleich CH oder CH₂ sind,
m und o unabhängig voneinander für 0 oder 1 stehen, mit der Maßgabe, dass
falls m für 0 steht, die gestrichelte Linie zwischen Y und dem benachbarten C Atom dann für eine Bindung steht und Y für CH oder ein trivalentes Heteroatom steht,
falls m für 1 steht, die gestrichelte Linie zwischen Y und dem benachbarten C Atom dann fehlt und Y für CH₂ oder ein divalentes Heteroatom steht,
falls o für 0 steht, die gestrichelte Linie zwischen X und dem benachbarten C Atom dann für eine Bindung steht und X für CH oder ein trivalentes Heteroatom steht,
falls o für 1 steht, die gestrichelte Linie zwischen X und dem benachbarten C Atom dann fehlt und X für CH₂ oder ein divalentes Heteroatom steht,
A für (CR₃R₄)ₚ und Q für (CR₉R₁₀)ₙ steht,
n und p unabhängig voneinander für 0 oder 1 stehen,
R₆, R₇, R₁₃ und R₁₄ unabhängig voneinander für Wasserstoff, Halogen, (C₁-C₄)-Alkyl, (C₁-C₄)-AlkylSO₂, SO₃H, Carboxy, (C₁-C₄)-Alkoxycarbonyl, (C₁-C₄)-Alkoxy, OH oder NR₁₅R₁₆ stehen,
R₁, R₂, R₃, R₄, R₉, R₁₀, R₁₁ und R₁₂ unabhängig voneinander für Wasserstoff, (C₁-C₄)-Alkyl, Carboxy, (C₁-C₄)-Alkoxycarbonyl oder (C₁-C₄)-Alkoxy stehen oder, falls X für CH oder CH₂ steht, dann R₁ und R₂ auch für OH oder NR₁₅R₁₆ stehen können oder, falls Y für CH oder CH₂ steht, dann R₁₁ und R₁₂ auch für CH oder NR₁₅R₁₆ stehen können,
R₅ , R₈ , R₁₅ und R₁₆ unabhängig voneinander für Wasserstoff, (C₁-C₄)-Alkyl, (C₁-C₄)-Alkoxy, R₁₇-O-C(O)-(C₁-C₄)-Alkyl oder (C₁-C₄)-Alkyl mit einer reaktiven Gruppe stehen, und
R₁₇ für Wasserstoff oder (C₁-C₄)-Alkyl steht,
in Form der freien Base oder in Form eines Säureadditionssalzes.

2. Verbindung der Formel I nach Anspruch 1 in freier Form oder in Form eines Säureadditionssalzes, worin X für O, S oder CH₂ steht und Y für O, S oder CH₂ steht, mit der Maßgabe, dass X und Y nicht beide zugleich für CH₂ stehen.

3. Verbindung der Formel I nach Anspruch 1, worin diese Verbindung ausgewählt ist aus
4,8-Dimethyl-2,3,4,9,10,11-hexahydro-1,6-dioxa-4,13-diaza-8-azoniapentacenchlorid,
8-Ethyl-4-methyl-2,3,4,9,1 0,11 -hexahydro-1,6-dioxa-4,13-diaza-8-azoniapentacenchlorid,
4,8-Dimethyl-3,8,9,10-tetrahydro-2H-1,6,11-trioxa-8,13-diaza-4-azoniapentacentetrafluorborat,
4,8-Dimethyl-2,3,9,10-tretrahydro-4H-1,6-dioxa-11-thia-4,13-diaza-8-azoniapentacenchlorid und
8-(3-Ethoxycarbonylpropyl)-4-methyl-3,8,9,10-tetrahydro-2H-1,6,11-trioxa-8,13-diaza-4-azoniapentacenchlorid.

4. Zusammensetzung, umfassend eine Verbindung nach einem der Ansprüche 1 bis 3 und einen pharmazeutisch annehmbaren Hilfsstoff oder ein pharmazeutisch annehmbares Verdünnungsmittel.

5. Verfahren zur Herstellung einer Verbindung der Formel I oder eines Salzes hiervon, umfassend die Stufen einer Umsetzung eines Phenolderivats der Formel III 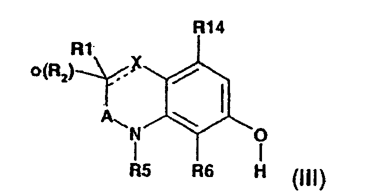 worin die Reste und Symbole A, X, R₁, R₂, R₅, R₆, R₁₄ und o die Bedeutungen haben, wie sie im Anspruch 1 für eine Verbindung der Formel I definiert sind, mit einer Nitrosoverbindung oder Diazoverbindung der Formel IV 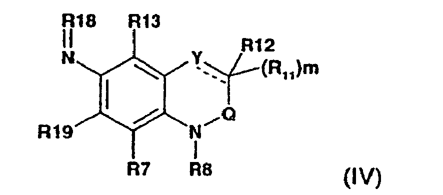 worin die Reste und Symbole Q, Y, R₇, R₈, R₁₁, R₁₂, R₁₃ und m die Bedeutungen haben, wie sie im Anspruch 1 für eine Verbindung der Formel I definiert sind, R₁₈ für Oxo oder p-Nitrophenyl-N= steht und R₁₉ für Hydroxy steht,
und die Gewinnung der erhaltenden Verbindung der Formel I in Form der freien Base oder in Form eines Säureadditionssalzes.

6. Verwendung einer Verbindung der Formel I nach einem der Ansprüche 1 bis 3 in Form der freien Base oder in Form eines Säureadditionssalzes als ein Abbildungsmittel im Nahen Infrarot (NIR).

7. Verwendung einer Verbindung der Formel II, 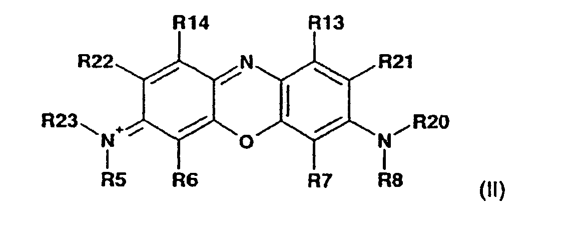 worin
R₆, R₇, R₁₃ und R₁₄ unabhängig voneinander für Wasserstoff, Halogen, (C₁-C₄)-Alkyl, (C₁-C₄)-AlkylSO₂, SO₃H, Carboxy, (C₁-C₄)-Alkoxycarbonyl, (C₁-C₄)-Alkoxy, OH oder NR₁₅R₁₆ stehen, und
R₂₁ und R₂₂ für Wasserstoff, (C₁-C₄)-Alkyl, (C₁-C₄)-Alkoxy, Phenyl, Phenylalkyl, Carboxy oder Halogen stehen,
R₁₄ und R₂₂ zusammen mit den Kohlenstoffatomen, an die sie gebunden sind, auch einen gesättigten oder ungesättigten Ring bilden können,
R₂₁ und R₁₃ zusammen mit den Kohlenstoffatomen, an die sie gebunden sind, auch einen gesättigten oder ungesättigten Ring bilden können,
R₅, R₈, R₂₀ und R₂₃ für Wasserstoff, (C₁-C₄)-Alkyl, (C₁-C₄)-Alkoxy, Polyoxyhydrocarbyl, Phenyl oder Phenylalkyl stehen,
R₈ und R₂₀ zusammen mit dem Stickstoffstoffatom, an das sie gebunden sind, einen gesättigten oder ungesättigten Ring bilden können,
R₂₃ und R₅ zusammen mit dem Stickstoffatom, an das sie gebunden sind, einen gesättigten oder ungesättigten Ring bilden können,
R₂₂ und R₂₃ zusammen mit den Atomen, an die sie gebunden sind, einen gesättigten oder ungesättigten Ring bilden können,
R₅ zusammen mit R₆ zusammen mit den Atomen, an die sie gebunden sind, einen gesättigten oder ungesättigten Ring bilden können,
R₇ zusammen mit R₈ zusammen mit den Atomen, an die sie gebunden sind, einen gesättigten oder ungesättigten Ring bilden können und
R₂₀ zusammen mit R₂₁ zusammen mit den Atomen, an die sie gebunden sind, einen gesättigten oder ungesättigten Ring bilden können,
wie in Anspruch 6 definiert als ein Abbildungsmittel im Nahen Infrarot.

8. Verwendung nach Anspruch 6 oder 7 als ein Abbildungsmittel im Nahen Infrarot zur Abbildung von Amyloidplaques.

9. Konjugat, umfassend eine Verbindung der Formel I nach einem der Ansprüche 1 bis 3, die über eine reaktive Gruppe kovalent an ein Biomolekül gebunden ist.

10. Konjugat nach Anspruch 9, worin das Biomolekül aus der Gruppe ausgewählt ist, die besteht aus einem Nucleosid, Nucleotid, Oligonucleotid, Nucleinsäure, Protein, Peptid, Aminosäure, Polysaccharid, Oligosaccharid, Monosaccharid, Arzneimittel oder einem kleinen Molekül mit einem Molekulargewicht von weniger als 500.

11. Konjugat nach Anspruch 9 oder 10, das zur Detektion unter Verwendung einer Strahlung im Nahen Infrarot befähigt ist.

## Revendications

1. Composés de formule I 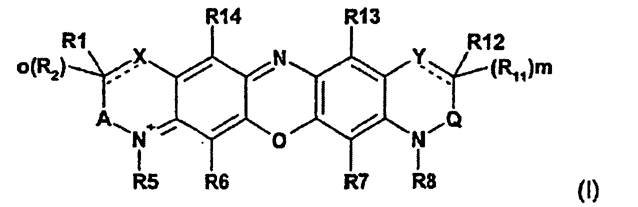 dans laquelle
X et Y représentent CH, CH₂ ou un hétéroatome divalent ou trivalent, à la condition que X et Y ne soient pas simultanément CH ou CH₂;
m et o représentent chacun indépendamment de l'autre 0 ou 1, à la condition que:
si m vaut 0, alors la ligne en tirets entre Y et l'atome de carbone voisin représente une liaison, et Y est CH ou un hétéroatome trivalent ;
si m vaut 1, alors la ligne en tirets entre Y et l'atome de carbone voisin est absente, et Y est CH₂ ou un hétéroatome divalent ;
si o vaut 0, alors la ligne en tirets entre X et l'atome de carbone voisin représente une liaison, et X est CH ou un hétéroatome trivalent ;
si o vaut 1, alors la ligne en tirets entre X et l'atome de carbone voisin est absente, et X est CH₂ ou un hétéroatome divalent ;
A représente (CR₃R₄)ₚ, et Q représente (CR₉R₁₀)ₙ ;
n et p valent chacun indépendamment de l'autre 0 ou 1;
R₆, R₇, R₁₃ et R₁₄ représentent chacun indépendamment de l'autre un atome d'hydrogène, un groupe halogéno, alkyle en C₁₋₄, (alkyle en C₁₋₄)SO₂, SO₃H, carboxy, (alcoxy en C₁₋₄)carbonyle, alcoxy en C₁₋₄, OH ou NR₁₅R₁₆ ;
R₁, R₂, R₃, R₄, R₉, R₁₀, R₁₁ et R₁₂ désignent chacun indépendamment des autres un atome d'hydrogène, un groupe alkyle en C₁₋₄, carboxy, (alcoxy en C₁₋₄)carbonyle ou alcoxy en C₁₋₄, ou bien, quand X est CH ou CH₂, alors R₁ et R₂ peuvent aussi être OH ou NR₁₅R₁₆, ou bien, quand Y est CH ou CH₂, alors R₁₁ et R₁₂ peuvent aussi être OH ou NR₁₅R₁₆ ;
R₅, R₈, R₁₅ et R₁₆ représentent chacun indépendamment des autres un atome d'hydrogène, un groupe alkyle en C₁₋₄, alcoxy en C₁₋₄, R₁₇O-C(O)-(alkyle en C₁-C₄) ou (groupe réactif)-(alkyle en C₁₋₄); et
R₁₇ est un atome d'hydrogène ou un groupe alkyle en C₁₋₄;
sous forme base libre ou sous forme d'un sel d'addition avec un acide.

2. Composé de formule I selon la revendication 1, sous forme base libre ou sous forme d'un sel d'addition avec un acide, dans lequel X est O, S ou CH₂, et Y est O, S ou CH₂, à la condition que X et Y ne soient pas tous les deux simultanément CH₂.

3. Composé de formule I selon la revendication 1, ledit composé étant choisi parmi
le chlorure de 4,8-diméthyl-2,3,4,9,10,11-hexahydro-1,6-dioxa-4,13-diaza-8-azonia-pentacène ;
le chlorure de 8-éthyl-4-méthyl-2,3,4,9,10,11-hexahydro-1,6-dioxa-4,13-diaza-8-azonia-pentacène ;
le tétrafluoroborate de 4,8-diméthyl-3,8,9,10-tétrahydro-2H-1,6,11-trioxa-8,13-diaza-4-azonia-pentacène ;
le chlorure de 4,8-diméthyl-2,3,9,10-tétrahydro-4H-1,6-dioxa-11-thia-4,13-diaza-8-azonia-pentacène; et
le chlorure de 8-(3-éthoxycarbonyl-propyl)-4-méthyl-3,8,9,10-tétrahydro-2H-1,6,11 -trioxa-8,13-diaza-4-azonia-pentacène.

4. Composition comprenant un composé selon l'une quelconque des revendications 1 à 3 et un excipient ou diluant acceptable d'un point de vue pharmaceutique.

5. Procédé de production d'un composé de formule I ou d'un sel de ce dernier, comprenant les étapes de réaction d'un dérivé du phénol de formule III 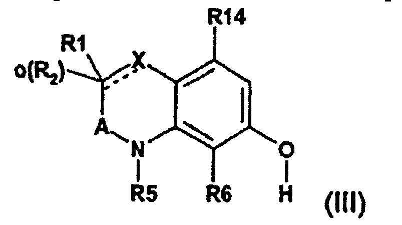 dans laquelle les radicaux et symboles A, X, R₁, R₂, R₅, R₆, R₁₄ et o ont les significations données dans la revendication 1 pour un composé de formule I, avec un composé nitroso ou diazo de formule IV 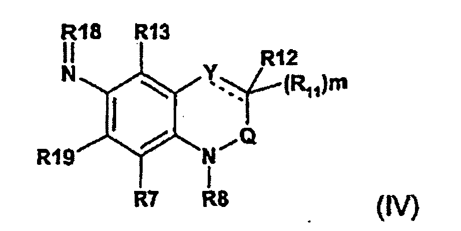 dans laquelle les radicaux et symboles Q, Y, R₇, R₈, R₁₁, R₁₂, R₁₃ et m ont les significations données dans la revendication 1 pour un composé de formule I, R₁₈ représente un radical oxo ou p-nitrophényl-N=, et R₁₉ est un groupe hydroxy ;
et de récupération du composé obtenu de formule I, sous forme base libre ou sous forme d'un sel d'addition avec un acide.

6. Utilisation d'un composé de formule I selon l'une quelconque des revendications 1 à 3 sous forme base libre ou sous forme d'un sel d'addition avec un acide, en tant qu'agent d'imagerie dans l'infrarouge proche.

7. Utilisation d'un composé de formule II 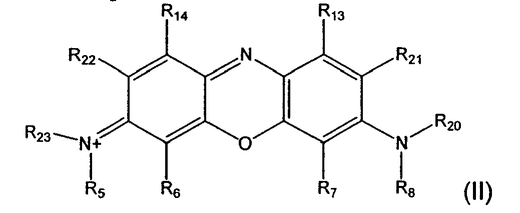 dans laquelle R₆, R₇, R₁₃ et R₁₄ représentent chacun indépendamment de l'autre un atome d'hydrogène, un groupe halogéno, alkyl en C₁₋₄, (alkyl en C₁-₄)SO₂, SO₃H, carboxy, (alkoxy en C₁₋₄)carbonyle, alkoxy en C₁₋₄, OH ou NR_{15,} R₁₆,
R₂₁ et R₂₂ représentent un atome d'hydrogène, un alkyl en C₁-₄, un alkoxy en C₁₋₄, un groupe phényle, phénylalkyle, carboxy ou halogéno;
R₁₄ et R₂₂ ensemble avec les atomes de carbone auxquels ils sont attachés peuvent également former un cycle saturé ou insaturé,
R₂₁ et R₁₃ ensemble avec les atomes de carbone auxquels ils sont attachés peuvent également former un cycle saturé ou insaturé,
R₅, R₈, R₂₀ et R₂₃ représentent un atome d'hydrogène, un groupe alkyle en C₁₋₄, alkoxy en C₁₋₄, polyoxyhydrocarbyl, phényle, phénylalkyle,
R₈ et R₂₀ ensemble avec l'atome d'azote auxquels ils sont attachés peuvent former un cycle saturé ou insaturé,
R₂₃ et R₅ ensemble avec l'atome d'azote auxquels ils sont attachés peuvent former un cycle saturé ou insaturé,
R₂₂ et R₂₃ ensemble avec les atomes auxquels ils sont attachés peuvent former un cycle saturé ou insaturé,
R₅ ensemble avec R₆ ensemble avec les atomes auxquels ils sont attachés peuvent former un cycle saturé ou insaturé,
R₇ ensemble avec R₈ ensemble avec les atomes auxquels ils sont attachés peuvent former un cycle saturé ou insaturé,
R₂₀ ensemble avec R₂₁, ensemble avec les atomes auxquels ils sont attachés peuvent former un cycle saturé ou insaturé,
tel que défini dans la revendication 6, en tant qu'agent d'imagerie dans l'infrarouge proche.

8. Utilisation selon la revendication 6 ou 7 en tant qu'agent d'imagerie dans l'infrarouge proche pour visualiser les plaques amyloïdes.

9. Conjugué comprenant un composé de formule I selon l'une quelconque des revendications 1-3, lié d'une manière covalente à une biomolécule par l'intermédiaire d'un groupe réactif.

10. Conjugué selon la revendication 9, dans lequel la biomolécule est choisie dans l'ensemble consistant en les nucléosides, les nucléotides, les oligonucléotides, les acides nucléiques, les protéines, les peptides, les acides aminés, les polysaccharides, les oligosaccharides, les monosaccharides, les médicaments ou les petites molécules ayant une masse moléculaire inférieure à 500.

11. Conjugué selon la revendication 9 ou 10, pouvant être détecté par utilisation d'un rayonnement infrarouge proche.
